# EUROPEAN PATENT APPLICATION

(11) **EP 2 767 272 A1**
(43) Date of publication of application: **20.08.2014**
(21) Application number: 13155649.0
(22) Date of filing: 18.02.2013
(51) Int. Cl.: A61K 9/20, A61K 31/535

(54) **Solid pharmaceutical dosage form of dolutegravir**

(71) Applicant: Ratiopharm GmbH, 89079 Ulm (DE)
(72) Inventor: Meergans, Dominique, 81379 München (DE); Prohl, Sabine, 81379 München (DE); Mika, Hans Juergen, 53229 Bonn (DE)
(74) Representative: Teipel, Stephan

(57) **Abstract**

The present invention relates to a solid pharmaceutical dosage form comprising dolutegravir, a method of its preparation and its use in the treatment of an HIV infection.

## Description

The present invention relates to a solid pharmaceutical dosage form comprising dolutegravir, a method of its preparation and its use in the treatment of an HIV infection.

Dolutegravir is the INN of (4R,12aS)-N-(2,4-difluorobenzyl)-7-hydroxy-4-methyl-6,8-dioxo-3,4,6,8,12,12a-hexahydro-2H-pyrido[1',2':4,5]pyrazino[2,1-b][1,3]oxazine-9-carboxamide which has the following chemical formula:

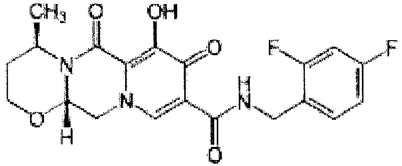

Dolutegravir is known from WO 2006/116764 as a compound possessing an antiviral activity, in particular an inhibitory activity against HIV integrase. WO 2006/116764 also discloses a tablet prepared using one among the many active ingredients disclosed in this document, microcrystalline cellulose, fumed silicon dioxide and stearic acid. The tablets are prepared by direct compression.

The sodium salt of dolutegravir and a specific crystalline form of this sodium salt or a hydrate thereof are disclosed in WO 2010/068253.

Dolutegravir is practically insoluble and even the known sodium salt of dolutegravir is practically insoluble (solubility below 0.1 mg/ml) in 0.1 N HCl (pH 1.2). At an increasing pH, for example to pH 6.8 in 50 mM KH₂PO₄ the solubility of the sodium salt of dolutegravir slightly increases but the salt still remains very slightly soluble (1-0.1 mg/ml), only. Due to this very low solubility of the active ingredient the tablets known from WO 2006/116764 are of little practical use because the active ingredient will hardly dissolve in the intestine resulting in a low bioavailability.

When repeating the preparation of the tablets disclosed in WO 2006/116764, it was furthermore observed that different formulation batches showed differences between the dissolution rates. Additionally, serious difficulties were observed due to an insufficient flowability of dolutegravir and its distinct tendency to sticking. Another disadvantage is the very high volume of this active pharmaceutical ingredient.

Therefore, there is a need for improved solid pharmaceutical dosage forms comprising dolutegravir. In particular, there is a need for solid pharmaceutical dosage forms comprising dolutegravir showing increased dissolution rate of the active ingredient. Furthermore, there is a need for solid pharmaceutical dosage forms comprising dolutegravir which can be manufactured in a reliable and repeatable manner.

It has now surprisingly been found that the above and other problems may be solved by the addition of a compound comprising an alkaline earth metal ion and/or an alkaline compound to the composition. Furthermore, it was surprisingly found that manufacturing of the pharmaceutical dosage form by wet granulation results in dosage forms of reliable and repeatable dissolution profile.

The present invention therefore relates to a solid pharmaceutical dosage form comprising dolutegravir or a pharmaceutically acceptable salt or solvate thereof and a compound comprising an alkaline earth metal ion and/or an alkaline compound. The invention further relates to a solid pharmaceutical dosage form comprising dolutegravir or a pharmaceutically acceptable salt or solvate thereof which is obtainable by wet granulation.

It has surprisingly been found that the addition of a compound comprising an alkaline earth metal ion and/or an alkaline compound results in a superior dissolution rate of the active from the solid pharmaceutical dosage form. Any alkaline earth metal is suitable in the compound comprising an alkaline earth metal ion. Particularly, magnesium and calcium ions are preferred. Therefore, the compound comprising an alkaline earth metal ion can be a magnesium or calcium compound. Also mixed compounds comprising both, magnesium and calcium ions or other ions such as alkali metal ions in addition to the alkaline earth metal ion are suitable. The magnesium or calcium compound may for example be selected from the group consisting of magnesium oxide, magnesium hydroxide, magnesium carbonate, magnesium chloride, magnesium sulfate, magnesium phosphate, calcium oxide, calcium hydroxide, calcium carbonate, calcium chloride, calcium sulfate, calcium phosphate and complexes of Mg²⁺ or Ca²⁺ with organic ligands, such as acetate, citrate or EDTA. Preferably, the compound comprising an alkaline earth metal ion comprises a magnesium ion. A preferred compound comprising an alkaline earth metal ion is magnesium oxide.

The solid pharmaceutical dosage form according to the invention may comprise an alkaline compound. In the context of the present invention an "alkaline compound" is defined as a compound which results in a pH above 7 when added to water. The alkaline compound can for example be a hydroxide or carbonate, such as a hydroxide or carbonate of an alkali metal or an alkaline earth metal. The alkaline compound can also be a salt of a weak acid, such as an alkali metal salt or an alkaline earth metal salt of a weak acid.

Preferably, the solid pharmaceutical dosage form according to the invention comprises a compound comprising an alkaline earth metal ion, in particular a magnesium ion, most preferably magnesium oxide.

The amount of the compound comprising an alkaline earth metal ion and/or an alkaline compound added to the dosage form is not particularly limited and can be selected by a person skilled in the art according to the requirements for example with respect to the desired dissolution rate or compressibility of prepared tablets. Suitably, dolutegravir or a pharmaceutically acceptable salt or solvate thereof and the compound comprising an alkaline earth metal ion and/or an alkaline compound are present in a molar ratio of from 1:10 to 10:1, preferably of from 1:5 to 5:1, more preferably of from 1:3 to 3:1 and most preferably of from 1:2 to 2:1.

A solid pharmaceutical dosage form may for example contain 1 to 10 % by weight of the compound comprising an alkaline earth metal ion and/or an alkaline compound based on the total weight of the dosage form, preferably from 2 to 6 % by weight, more preferably from 3 to 5 % by weight. The total weight of the dosage form is considered as the total weight of for example a tablet without coating or the total weight of a capsule fill without the weight of the capsule shell.

The solid pharmaceutical dosage form may comprise one or more further pharmaceutically acceptable excipients, such as e.g. fillers, binder, disintegrants, glidants, surfactants and flow regulators ("Lexikon der Hilfsstoffe für Pharmazie, Kosmetik und angrenzende Gebiete", edited by H. P. Fiedler, 5th Edition, and "Handbook of Pharmaceutical Excipients", 6th Edition, edited by Arthur H. Kibbe, American Pharmaceutical Association, Washington, USA, and Pharmaceutical Press, London).

Fillers: The pharmaceutical dosage form can contain one or more filler(s). In general, a filler is a substance that increases the bulk volume of the mixture and thus the size of the resulting pharmaceutical dosage form. Preferred examples of fillers are selected from sugar, microcrystalline cellulose, lactose starch and mixtures thereof. The filler may be present in a proportion of 0 to 80% by weight, preferred between 10 and 60% by weight of the total weight of the dosage form.

Binders: Binders are adhesives to promote size enlargement to produce granules and thus improve flowability of the blend during the manufacturing process. Binders may also improve the hardness of the tablets by enhancing intragranular as well as intergranular forces. Preferred binders for wet granulation are povidone, tragacanth, sodium alginate, gum arabic, starch pregelatinized, gelatin and cellulosic derivates. The dosage form of the invention may, for example, comprise the following hydrophoilic poymers as binder: polysaccharides, such as hydroxypropyl methyl cellulose (HPMC) e.g. Pharmacoat 603^{®}. Typically, the binder is present in an amount of 0 to 40% by weight, preferably between 2 and 10% by weight of the total weight of the pharmaceutical dosage form.

Lubricants: Lubricants are agents which act on the flowability of the powder by reducing interparticle friction and cohesion to be compressed. Suitable lubricants colloidal silicon dioxide, such as aerosol, talc, stearic acid, magnesium stearate, calcium stearate, glyceryl behenate, sodium stearyl fumarate and silica gel. Typically, the lubricant is present in an amount of 0 to 5% by weight, preferably between 0.1 and 3% by weight of the total weight of the pharmaceutical dosage form.

Surfactant: The term "surfactant" refers to an excipient that lowers the surface tension of a liquid. Examples of surfactant include tween 80, polyoxyethylene-polyoxypropylene copolymer and sodium lauryl sulfate. Typically, the surfactant is present in an amount of 0 to 2% by weight, preferably between 0.4 and 1% by weight of the total weight of the pharmaceutical dosage form.

Disintegrants: The term "disintegrant" refers to an excipient which expands and dissolve when wet causing the tablet to break apart in the digestive tract, releasing the active ingredients for absorption. Preferred disintegrants are croscarmellose sodium (e.g. Ac-Di-Sol^{®}), sodium carboxymethyl starch, cross-linked polyvinylpyrrolidone (crospovidon), sodium carboxymethyl glycolate and sodium bicarbonate. Typically, the disintegrant is present in an amount of 0 to 30% by weight, preferably between 3 and 15% by weight of the total weight of the pharmaceutical dosage form.

Flow regulators: As flow regulator there can be used e.g. colloidal silica (e.g. Aerosil^{®}). Preferably the flow regulator is present in an amount of 0 to 5% by weight, more preferably in an amount between 1 and 4% by weight of the total weight of the dosage form.

In a preferred embodiment the solid pharmaceutical dosage form of the present invention comprises at least one disintegrant, such as crosscarmelose sodium, for example in an amount of from 3 to 6 % by weight of the total weight of the dosage form.

The solid pharmaceutical dosage form of the invention preferably is an oral dosage form, such as capsules, tablets, pellets or sachets. Tablets are particularly preferred. The tablets may or may not have a coating.

In a further embodiment the solid pharmaceutical dosage form of the present invention is obtainable by wet granulation. In the preparation by wet granulation preferably water is used as granulation liquid. The preparation of the solid pharmaceutical dosage form by wet granulation has the advantage that disadvantages of dolutegravir regarding insufficient flowability and the tendency to sticking as well as disadvantages associated with the very high volume of the active are overcome. The granules obtained by wet granulation can either be used for the preparation of capsules, they can be filled into sachets or they can be compressed into tablets.

In the solid pharmaceutical dosage form according to the invention the dissolution rate of the active is significantly increased. The dissolution rate can be measured using USP paddle Apparatus II in 900 ml 0.1 N HCl, pH 1.2, at 37°C and 100 rpm, increasing the rotation speed to 150 rpm after 90 minutes.

The present invention therefore also relates to a solid pharmaceutical dosage form comprising dolutegravir or a pharmaceutically acceptable salt or solvate thereof, from which at least 10 % of the total amount of the dolutegravir present in the dosage form are dissolved in less than 10 minutes when measured using the above described method. Preferably, at least 70 % of the total amount of dolutegravir present in the dosage form are dissolved in less than 20 minutes. These solid pharmaceutical dosage forms may comprise a compound comprising an alkaline earth metal ion and/or an alkaline compound, in particular magnesium oxide and/or may be obtainable by wet granulation and furthermore can make use of all of the preferred embodiments described above.

The dolutegravir can be present in the solid pharmaceutical dosage form of the present invention either in its free form or in the form of a pharmaceutically acceptable salt or solvate thereof. Pharmaceutically acceptable salts or solvates are known to a person skilled in the art and can be prepared for example by the addition of known acids or bases. Preferably, the dolutegravir is present as dolutegravir sodium salt. As solvate the hydrate is preferred.

Advantageously, the dolutegravir sodium salt is crystalline.

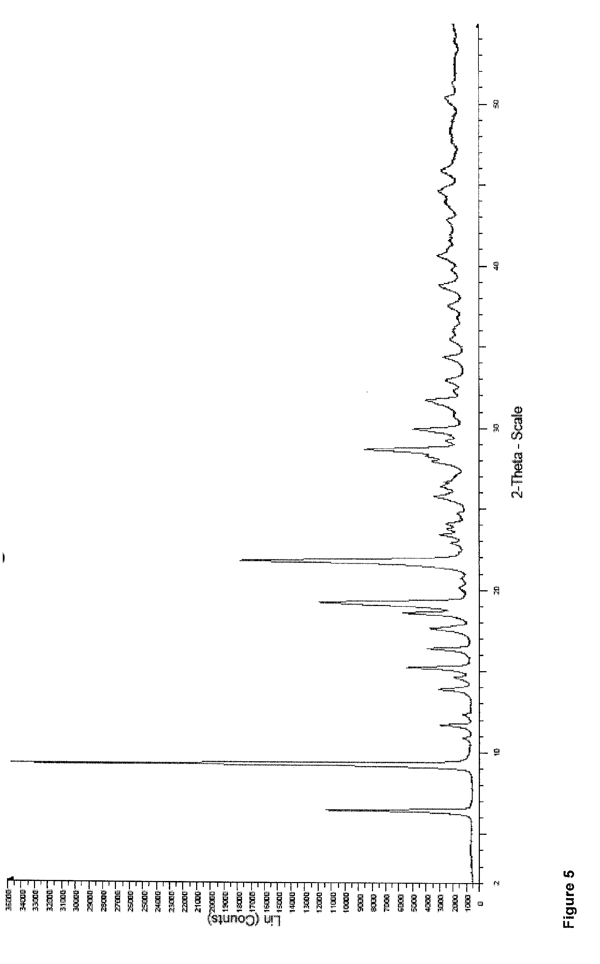

In one embodiment, the solid pharmaceutical dosage form of the present invention contains a crystalline form of dolutegravir sodium salt which has diffraction peaks in an X-ray powder diffraction pattern at 6.4 ± 0.2 and 9.2 ± 0.2 degrees 2-theta, preferably at 6.4 ± 0.2, 9.2 ± 0.2 and 13.8 ± 0.2 degrees 2-theta, more preferably at 6.4 ± 0.2, 9.2 ± 0.2, 13.8 ± 0.2 and 19.2 ± 0.2 degrees 2-theta, even more preferably at 6.4 ± 0.2, 9.2 ± 0.2, 13.8 ± 0.2, 19.2 ± 0.2 and 21.8 ± 0.2 degrees 2-theta and most preferably at 6.4 ± 0.2, 9.2 ±0.2, 13.8 ± 0.2, 14.6 ± 0.2, 15.2 ± 0.2, 17.6 ± 0.2, 19.2 ± 0.2, 21.8 ± 0.2, 24.1 ± 0.2 and 28.7 ± 0.2 degrees 2-theta. The crystalline form of the dolutegravir sodium salt in a particularly preferred embodiment has an X-ray powder diffraction pattern substantially as shown in Figure 5 when measured using Cu k alpha radiation.

The preparation of crystalline dolutegravir sodium salt and the above described polymorph of this salt is disclosed in WO 2010/068253.

The present invention furthermore relates to a method of preparation of a solid pharmaceutical dosage form as described above, the method comprising the steps of mixing dolutegravir or a pharmaceutically acceptable salt or solvate thereof with a compound comprising an alkaline earth metal ion and/or an alkaline compound and optionally further ingredients, and wet granulation of the obtained mixture. The method may further comprise the step of compressing the obtained granules.

Finally, the present invention also relates to the above described solid pharmaceutical dosage form for use in a method of treatment of a HIV infection in a human.

In the attached figures
Figure 1 shows the dissolution profiles of solid pharmaceutical dosage forms according to the invention (Example 1 - tablet) and according to the prior art,
Figure 2 shows the dissolution profiles of solid pharmaceutical dosage forms according to the invention (Example 1 - granules) and according to the prior art,
Figure 3 shows the dissolution profiles of solid pharmaceutical dosage forms according to the invention (Example 2 - tablet) and according to the prior art,
Figure 4 shows the dissolution profiles of solid pharmaceutical dosage forms according to the invention (Example 3 - tablet) and according to the prior art, and
Figure 5 shows the X-ray powder diffraction pattern of dolutegravir sodium salt.

The invention will now be further explained by way of examples which are not intended as limiting.

### Example 1

Tablets were prepared using the ingredients as summarized in the following table 1.

**Table 1**

| **Compound** | **Brand ®** | **amount [mg]** | **amount [%]** |
|---|---|---|---|
| ***intragranular*** | | | |
| Dolutegravir sodium | - | 50.00* | 21.11 |
| HPMC | Pharmacoat 603 | 8.00 | 3.38 |
| Lactose monohydrate | FlowLac 100 (spray dried) | 60.00 | 25.33 |
| Croscarmellose sodium | Ac-Di-Sol | 5.00 | 2.11 |
| Microcrystalline cellulose | Avicel PH 101 | 50.00 | 21.11 |
| Sodium lauryl sulphate (SDS) | - | 2.00 | 0.84 |
| Magnesium oxide | - | 8.00 | 3.38 |

| ***extragranular*** | | | |
|---|---|---|---|
| Magnesium stearate | - | 2.90 | 1.22 |
| Croscarmellose sodium | Ac-Di-Sol | 5.00 | 2.11 |
| Microcrystalline cellulose + Colloidal silica | Prosolv SMCC 90 | 46.00 | 19.42 |
| | **TOTAL** | **236.90** | **100.00** |

| | | | |
|---|---|---|---|
| *based on the free acid of dolutegravir | | | |

### Manufacturing description

An aqueous solution comprising Pharmacoat 603 and Magnesium oxide was prepared. Agitation was required in order to achieve complete suspension. Dolutegravir sodium, SDS, FlowLac 100, Prosolv SMCC 90 and half the amount of Ac-Di-Sol were granulated with the before prepared solution. The obtained granules were sieved through 2000µm mesh size and subsequently dried at 40°C. After sieving the dried granules through 630µm mesh size remaining quantity of Ac-Di-Sol and Prosolv SMCC 90 were added. The mixture was blended for 10 minutes in a Turbula T10B tumble mixer. After addition of Magnesium stearate blending was continued for further 3 minutes. Finally, the blend was compressed into tablets on an eccentric press (e.g. Korsch Ek0) with a 9 mm biconvex tablet punch.

### Example 2

Tablets were prepared using the ingredients as summarized in the following table 2.

**Table 2**

| **Compound** | **Brand ®** | **amount [mg]** | **amount [%]** |
|---|---|---|---|
| ***intragranular*** | | | |
| Dolutegravir sodium | - | 50.00* | 21.11 |
| HPMC | Pharmacoat 603 | 8.00 | 3.38 |
| Microcrystalline cellulose + Colloidal silica | Prosolv SMCC 90 | 120.00 | 50.65 |
| Croscarmellose sodium | Ac-Di-Sol | 5.00 | 2.11 |
| Sodium lauryl sulphate (SDS) | - | 2.00 | 0.84 |
| Magnesium oxide | - | 8.00 | 3.38 |

| ***extragranular*** | | | |
|---|---|---|---|
| Magnesium stearate | - | 2.90 | 1.22 |
| Croscarmellose sodium | Ac-Di-Sol | 5.00 | 2.11 |
| Microcrystalline cellulose + Colloidal silica | Prosolv SMCC 90 | 36.00 | 15.20 |
| | **TOTAL** | **236.90** | **100.00** |

| | | | |
|---|---|---|---|
| *based on the free acid of dolutegravir | | | |

### Manufacturing description

An aqueous solution comprising Pharmacoat 603 and Magnesium oxide was prepared. Agitation was required in order to achieve complete suspension. Dolutegravir sodium, SDS, Prosolv SMCC 90 and half the amount of Ac-Di-Sol were granulated with the before prepared solution. The obtained granules were sieved through 2000µm mesh size and subsequently dried at 40°C. After sieving the dried granules through 630µm mesh size remaining quantity of Ac-Di-Sol and Prosolv SMCC 90 were added. The mixture was blended for 10 minutes in a Turbula T10B tumble mixer. After addition of Magnesium stearate blending was continued for further 3 minutes. Finally, the blend was compressed into tablets on an eccentric press (e.g. Korsch Ek0) with a 9 mm biconvex tablet punch.

### Example 3

Tablets were prepared using the ingredients as summarized in the following table 3.

**Table 3**

| **Compound** | **Brand ®** | **amount [mg]** | **amount [%]** |
|---|---|---|---|
| ***intragranular*** | | | |
| Dolutegravir sodium | - | 50.00* | 21.11 |
| HPMC | Pharmacoat 603 | 8.00 | 3.38 |
| Lactose monohydrate | Granulac 200 (milled Lactose) | 60.00 | 25.33 |
| Microcrystalline cellulose | Avicel PH 101 | 50.00 | 21.11 |
| Croscarmellose sodium | Ac-Di-Sol | 5.00 | 2.11 |
| Sodium lauryl sulphate (SDS) | - | 2.00 | 0.84 |
| Magnesium oxide | - | 8.00 | 3.38 |

| ***extragranular*** | | | |
|---|---|---|---|
| Magnesium stearate | - | 2.90 | 1.22 |
| Croscarmellose sodium | Ac-Di-Sol | 5.00 | 2.11 |
| Microcrystalline cellulose + Colloidal silica | Prosolv SMCC 90 | 46.00 | 15.20 |
| | **TOTAL** | **236.90** | **100.00** |

| | | | |
|---|---|---|---|
| *based on the free acid of dolutegravir | | | |

### Manufacturing description

An aqueous solution comprising Pharmacoat 603 and Magnesium oxide was prepared. Agitation was required in order to achieve complete suspension. Dolutegravir sodium, SDS, Granulac 200 and half the amount of Ac-Di-Sol were granulated with the before prepared solution. The obtained granules were sieved through 2000µm mesh size and subsequently dried at 40°C. After sieving the dried granules through 630µm mesh size remaining quantity of Ac-Di-Sol and Prosolv SMCC 90 were added. The mixture was blended for 10 minutes in a Turbula T10B tumble mixer. After addition of Magnesium stearate blending was continued for further 3 minutes. Finally, the blend was compressed into tablets on an eccentric press (e.g. Korsch Ek0) with a 9 mm biconvex tablet punch.

### Example 4

Tablets were prepared using the ingredients as summarized in the following table 4.

**Table 4**

| **Compound** | **Brand ®** | **amount [mg]** | **amount [%]** |
|---|---|---|---|
| ***intragranular*** | | | |
| Dolutegravir sodium | - | 50.00* | 21.11 |
| HPMC | Pharmacoat 603 | 8.00 | 3.38 |
| Lactose monohydrate | Granulac 200 (milled Lactose) | 56.00 | 23.64 |
| Microcrystalline cellulose | Avicel PH 101 | 50.00 | 21.11 |
| Croscarmellose sodium | Ac-Di-Sol | 5.00 | 2.11 |
| Sodium lauryl sulphate (SDS) | - | 2.00 | 0.84 |
| Calcium carbonate | - | 12.00 | 5.07 |

| ***extragranular*** | | | |
|---|---|---|---|
| Magnesium stearate | - | 2.90 | 1.22 |
| Croscarmellose sodium | Ac-Di-Sol | 5.00 | 2.11 |
| Microcrystalline cellulose + Colloidal silica | Prosolv SMCC 90 | 46.00 | 19.42 |
| | **TOTAL** | **236.90** | **100.00** |

| | | | |
|---|---|---|---|
| *based on the free acid of dolutegravir | | | |

### Manufacturing description

An aqueous solution comprising Pharmacoat 603 and Calcium carbonat was prepared. Agitation was required in order to achieve complete suspension. Dolutegravir sodium, SDS, Granulac 200 and half the amount of Ac-Di-Sol were granulated with the before prepared solution. The obtained granules were sieved through 2000µm mesh size and subsequently dried at 40°C. After sieving the dried granules through 630µm mesh size remaining quantity of Ac-Di-Sol and Prosolv SMCC 90 were added. The mixture was blended for 10 minutes in a Turbula T10B tumble mixer. After addition of Magnesium stearate blending was continued for further 3 minutes. Finally, the blend was compressed into tablets on an eccentric press (e.g. Korsch Ek0) with a 9 mm biconvex tablet punch.

### Comparative Example (according to Example 2 of WO 2006/116764)

**Tablet mixture**

| **Compound** | **Brand ®** | **amount [mg]** | **amount [%]** |
|---|---|---|---|
| Dolutegravir sodium | - | 50.00* | 37.5 |
| Microcrystalline cellulose | Avicel PH 101 | 80.00 | 60.2 |
| Silicon dioxide | Aerosil | 2.00 | 1.5 |
| Stearic acid | - | 1.00 | 0.8 |
| | **TOTAL** | **133.00** | **100.00** |

| | | | |
|---|---|---|---|
| *based on the free acid of dolutegravir | | | |

All ingredients are mixed together in order to get tablet mixture.

The tablet mixture was compressed in order to get tablets.

### Example 5

The dissolution profiles of the tablets obtained in examples 1, 2 and 3 as well as the dissolution profile of the granules used in example 1 were measured according to the above described method. For comparison, also the dissolution profile of the tablet obtained in the above comparative example according to example 2 of WO 2006/116764 and the dissolution profile of the mixture used in the above preparation of the tablet according to example 2 of WO 2006/116764 were also measured according to the above method. However, in order to avoid a possible influence of the salt of dolutegravir on the dissolution rate dolutegravir sodium salt was used in both, the examples according to the invention and the comparative example.

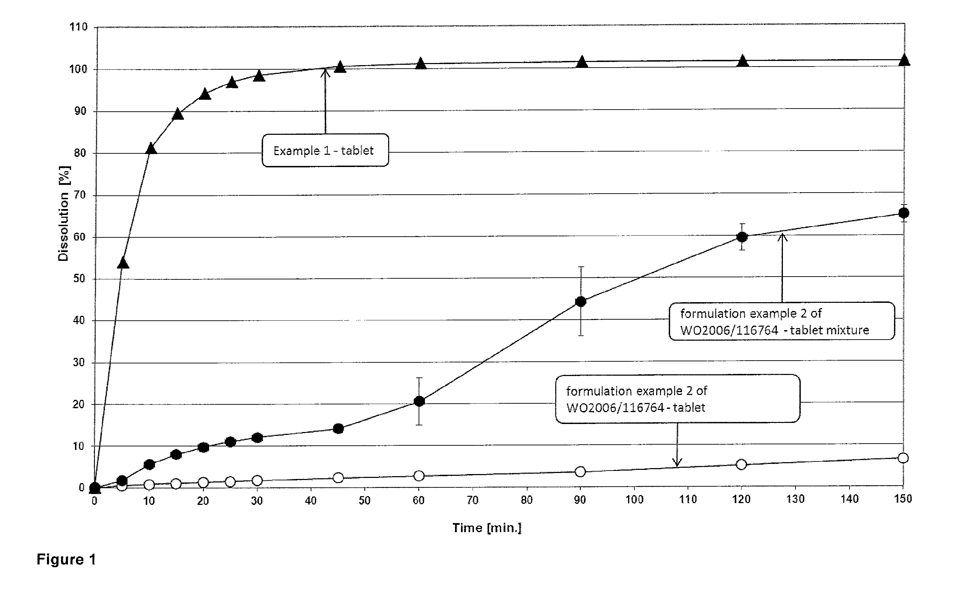

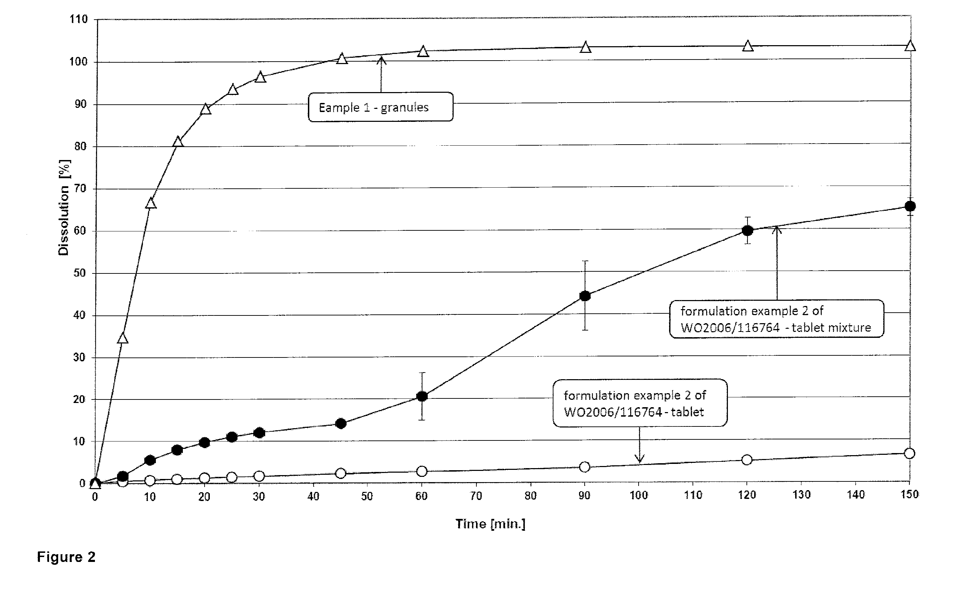

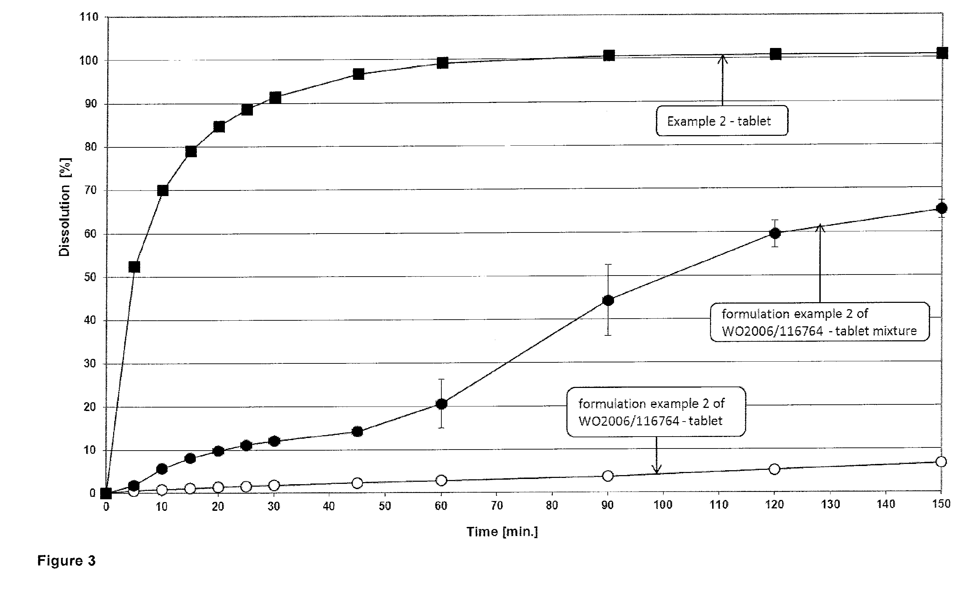

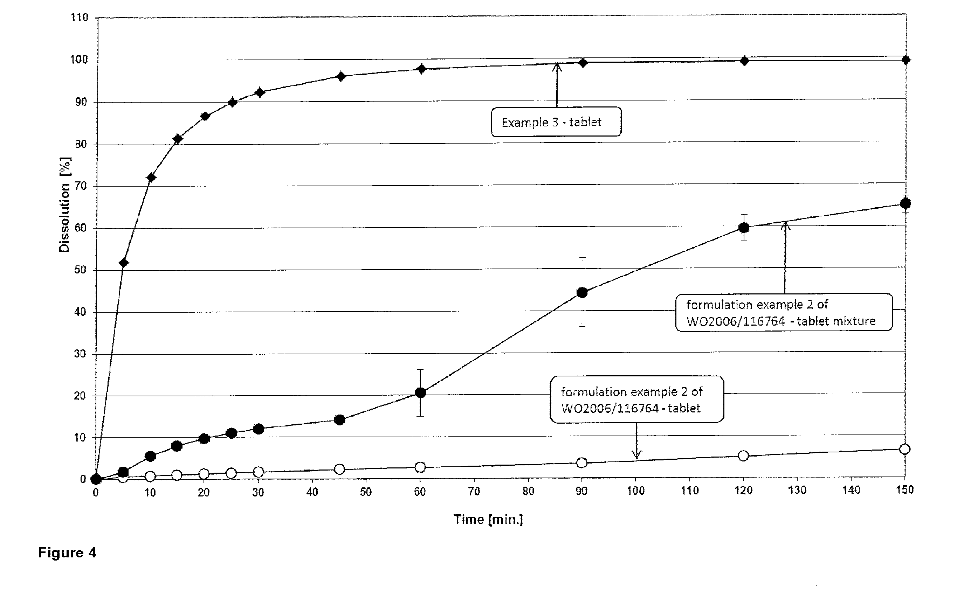

The results of the dissolution tests are summarized in attached Figures 1 to 4. It can be seen that the dissolution rate of the active is significantly increased in the mixture and tablets of the present invention compared to the prior art tablet and the mixture used in the preparation of the prior art tablet.

Furthermore, the prior art formulations have been prepared twice. Both batches were analyzed by the dissolution testing. It can be seen that both formulation batches showed significant differences between the dissolution rates. Thus, the prior art formulations do not provide repeatable and reliable dissolution.

## Claims

1. Solid pharmaceutical dosage form comprising dolutegravir or a pharmaceutically acceptable salt or solvate thereof and a compound comprising an alkaline earth metal ion and/or an alkaline compound.

2. Solid pharmaceutical dosage form according to claim 1, wherein the compound comprising an alkaline earth metal ion is a magnesium or calcium compound.

3. Solid pharmaceutical dosage form according to claim 2, wherein the magnesium or calcium compound is selected from the group consisting of magnesium oxide, magnesium hydroxide, magnesium carbonate, magnesium chloride, magnesium sulfate, magnesium phosphate, calcium oxide, calcium hydroxide, calcium carbonate, calcium chloride, calcium sulfate, calcium phosphate and complexes of Mg²⁺ or Ca²⁺ with organic ligands.

4. Solid pharmaceutical dosage form according to any of the preceding claims, wherein the alkaline compound is a hydroxide or carbonate, preferably a hydroxide or carbonate of an alkali metal or an alkaline earth metal.

5. Solid pharmaceutical dosage form according to claim 1, comprising dolutegravir or a pharmaceutically acceptable salt or solvate thereof and the compound comprising an alkaline earth metal ion and/or an alkaline compound in a molar ratio of from 1:10 to 10:1.

6. Solid pharmaceutical dosage form according to any of the preceding claims, containing from 1 to 10 % by weight of the compound comprising an alkaline earth metal ion and/or an alkaline compound based on the total weight of the dosage form.

7. Solid pharmaceutical dosage form according to any of the preceding claims comprising one or more further pharmaceutically acceptable excipients.

8. Solid pharmaceutical dosage form according to claim 7, comprising at least one disintegrant.

9. Solid pharmaceutical dosage form according to any of the preceding claims which is in the form of a capsule or tablet.

10. Solid pharmaceutical dosage form according to claim 9 which is obtainable by wet granulation.

11. Solid pharmaceutical dosage form comprising dolutegravir or a pharmaceutically acceptable salt or solvate thereof which is obtainable by wet granulation.

12. Solid pharmaceutical dosage form comprising dolutegravir or a pharmaceutically acceptable salt or solvate thereof, from which at least 10 % of the total amount of the dolutegravir present in the dosage form are dissolved in less than 10 minutes when measured using USP paddle Apparatus II in 900 ml 0.1 N HCl, pH 1.2, at 37°C and 100 rpm.

13. Solid pharmaceutical dosage form according to claim 12 from which at least 70 % of the total amount of dolutegravir present in the dosage form are dissolved in less than 20 minutes when measured using USP paddle Apparatus II in 900 ml 0.1 N HCl, pH 1.2, at 37°C and 100 rpm.

14. Solid pharmaceutical dosage form according to any of the preceding claims, wherein the dolutegravir is present as dolutegravir sodium salt.

15. Solid pharmaceutical dosage form according to claim 14, wherein the dolutegravir sodium salt is crystalline.

16. Solid pharmaceutical dosage form according to claim 15, wherein the crystalline form of the dolutegravir sodium salt has diffraction peaks in a X-ray powder diffraction pattern at 6.4 ± 0.2 and 9.2 ± 0.2 degrees 2-theta.

17. Method of preparation of a solid pharmaceutical dosage form according to any of the preceding claims, the method comprising the steps of mixing dolutegravir or a pharmaceutically acceptable salt or solvate thereof with a compound comprising an alkaline earth metal ion and/or an alkaline compound and optionally further ingredients; and wet granulation of the obtained mixture.

18. Solid pharmaceutical dosage form according to any of claims 1-16 for use in a method of treatment of an HIV infection in a human.
